# FASCICULE DE BREVET EUROPEEN

(11) **EP 1 388 546 B1**
(45) Date de publication et mention de la délivrance du brevet: **08.09.2010**
(21) Numéro de dépôt: 03292375.7
(22) Date de dépôt: 27.07.1998
(51) Int. Cl.: C08F 4/00, C07B 37/02, C07B 33/00, C07B 35/02, C07B 37/04, C07B 37/12

(54) **Utilisation des composés ioniques ayant une charge anionique délocalisée comme composants de catalyseur**
Verwendung von ionenischen Verbindungen mit einer delokalisierten anionischen Ladung als Katalysatoren
Use of ionic compounds having a delocalised anionic charge as catalyst components

(30) Priorité: 25.07.1997 CA 2211465
(43) Date de publication de la demande: 11.02.2004
(62) Demande divisionnaire de: 98941464.4
(73) Titulaire: ACEP Inc., Montréal, Quebec H2Z 1A4 (CA); UNIVERSITE DE MONTREAL, Montréal, Québec H3C 3J7 (CA); CENTRE NATIONAL DE LA RECHERCHE SCIENTIFIQUE (CNRS), 75016 Paris (FR); Institute of Organic Chemistry, Kiev-94, 253660 (UA)
(72) Inventeur: Armand, Michel, Montréal Québec (CA); Michot, Christophe, Montréal Québec (CA); Yagupolskii, Yurii, Kiev, 252005 (UA); Yagupolskii, Lev, Kiev, 252042 (UA); Bezdudny, Andrej, Kiev, 252057 (UA); Kondratenko, Natalya, Kiev, 253154 (UA)
(74) Mandataire: Sueur, Yvette

(56) Documents cités:
- J.A. BOON, ET AL.: "Friedel-Crafts reactions in ambient-temperature molten salts" JOURNAL OF ORGANIC CHEMISTRY, vol. 51, no. 4, 21 février 1986 (1986-02-21), pages 480-483, XP000943014 AMERICAN CHEMICAL SOCIETY, WASHINGTON, DC, US ISSN: 0022-3263
- D.A. JAEGER, ET AL.: "Diels-Alder reactions in ethylammonium nitrate, a low melting fused salt" TETRAHEDRON LETTERS, vol. 30, no. 14, 1989, pages 1785-1788, XP002265852 ELSEVIER SCIENCE PUBLISHERS, AMSTERDAM, NL

## Description

La présente invention a pour objet l'utilisation de composés ioniques comprenant une charge anionique fortement délocalisée en tant que catalyseur pour une réaction de polymérisation, une réaction de condensation, une réaction d'addition ou d'élimination, une réaction d'oxydation ou de réduction, une solvolyse, une réaction de Friedel et Craft ou une réaction de Diels Alder comme définie dans la revendication 1.

Il est bien connu que les sels des acides forts de type HClO₄, HBF₄, HPF₆, HR_{F}SO₃, (R_{F} radical perfluoré) présentent des propriétés dans le domaine de l'électrochimie et de la catalyse, mais que ces propriétés sont limitées. On connaît par ailleurs les "superacides" obtenus par addition d'un acide de Lewis tel que SbF₅ aux composés précités. Ces composés ne sont cependant pas stables autrement que sous forme protonée et dans des milieux non solvatants tels que les hydrocarbures aliphatiques. Les sels sont instables dans les solvants polaires usuels.

Depuis peu, les dérivés des perfluorosulfonimides H[R_{F}SO₂NSO₂R_{F}] (R_{F} = perfluoroalkyle) ont été étudiés. Ils présentent des propriétés de stabilité intéressantes sous forme protonée ou sous forme de sels et sont utilisés comme solutés dans l'électrochimie et comme catalyseurs. Toutefois, il n'est pas possible de conférer à ces sels toutes les propriétés requises pour toutes les applications, en particulier en termes d'acidité, de dissociation ou de solubilité, car l'utilisation de composés contenant des chaînes perfluorées de plusieurs carbones n'augmente que peu l'acidité ou la dissociation des sels, par rapport au composé le plus simple R_{F} = CF₃, et induit une rigidité de la molécule au détriment des propriétés de conductivité. Les longues chaînes fluorées sont à la fois hydrophobes et oléophobes et ne permettent pas d'augmenter la solubilité dans les milieux organiques d'une façon notable. Le remplacement de groupements R_{F} dans les imides simples par des groupements non perfluorés ou seulement partiellement fluorés diminue l'acidité et la solubilité d'une façon sensible.

La publication de Jaeger et al. (Tetrahedron Letters, 1989, 30(14), 1785-1788) décrit l'utilisation de nitrate d'éthylammonium sous forme de sel fondu comme solvant pour des réactions de Diels-Alder. La publication de Boon et al. (J. Org. Chem., 1986, 51, 480-483) décrit des réactions de Friedel-Crafts dans des sels fondus à température-ambiante, les sels étant des mélanges de chlorure de 1-méthyl-3-éthylimidazolium.

Le but de la présente invention est de fournir de nouveaux composés ioniques dérivés de perfluorosulfonimides dans lesquels la délocalisation de la charge anionique est améliorée, ce qui entraîne une acidité et une dissociation nettement supérieures à celles des composés connus, tout en conservant une bonne stabilité et utilisables comme catalyseur.

C'est pourquoi la présente invention a pour objet l'utilisation d'un composé ionique en tant que catalyseur pour une réaction de polymérisation, une réaction de condensation, une réaction d'addition ou d'élimination, une réaction d'oxydation ou de réduction, une solvolyse, une réaction de Friedel et Craft ou une réaction de Diels Alder" **caractérisée en ce que** ledit composé ionique répond à l'une des formules suivantes : 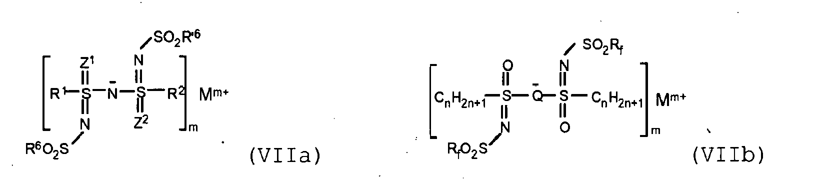 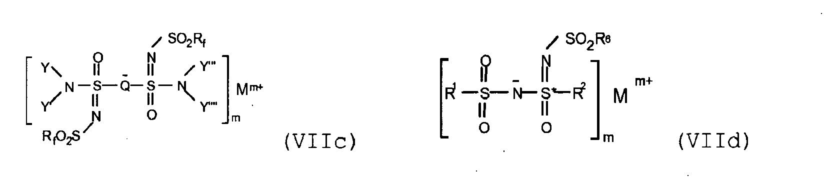 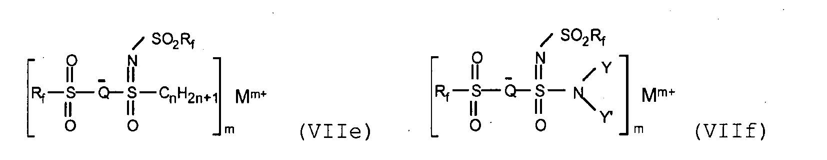 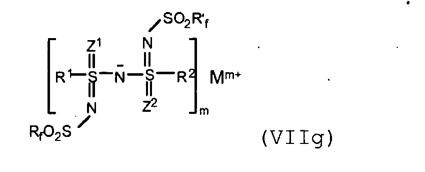 dans lesquelles :
- M^{m+} est le proton, ou un cation métallique ayant la valence m choisi parmi les ions des métaux alcalins, des métaux alcalino-terreux, des métaux de transition, des terres rares, ou un cation organique onium, ou un cation organo-métallique, 1 ≤ m ≤ 3 ;
- Q représente N, CR⁵, CCN ou CSO₂R⁵ ;
- Z¹ et Z² désignés ci-après par Zⁱ, représentent indépendamment les uns des autres =O, =NC≡N, =C(C≡N)₂, =NS(=Z)₂R⁶ ou =C[S(=Z)₂R⁶]₂;
- R¹, R², et R⁶, désignés ci-après par Rⁱ, représentent indépendamment les uns des autres Y, YO-, YS-, Y₂N- ou F ;
- Y représente un radical organique monovalent, ayant de 1 à 16 atomes de carbone, choisi parmi les radicaux alkyles, alkényles, oxa-alkyles, oxa-alkényles, aza-alkyles, aza-alkényles, aryles ou alkylaryles, perfluoroalkyles, ou parmi les radicaux obtenus à partir des radicaux précités par substitution, dans les chaînes et / ou la partie aromatique, par des hétéroatomes tels que les halogènes, l'oxygène, l'azote, le soufre, le phosphore, étant entendu que si le soufre ou le phosphore sont présents, ils peuvent éventuellement être liés à des atomes d'oxygène ou d'azote substitués, ou bien Y est une unité de répétition d'une trame polymérique ;
- dans les formules (VIIb), (VIIc) (VIIe), (VIIf) et (VIIg), R_{f} représente un radical perfluoroalkyle ;
- dans les formules (VIIb) et (VIIe), CₙH₂ₙ₊₁ réprésente un radical alkyle ;
- dans les formules (VIIc) et (VIIf), Y', Y" et Y" ont la même définition que Y ;
- dans la formule (VIIa),R'⁶ est choisi dans le même groupe que R⁶ ;
- dans la formule (VIId), R⁶ est différent de R¹.

Lorsque M^{m+} est un cation métallique, il peut être choisi parmi Li⁺ et K⁺, Mg⁺⁺, Ca⁺⁺ ou Ba⁺⁺, Cu⁺⁺, Zn⁺⁺, Fe⁺⁺ ou Re⁺⁺⁺.

Lorsque M^{m+} est un cation onium, il peut être choisi parmi les ions ammonium [N(Y^{j})₄]⁺, les ions amidinium RC[N(Y^{j})₂]⁺, les ions guanidinium C[N(Y^{j})₂]₃⁺, les ions pyridinium C₅[N(Y^{j})₆]', les ions imidazolium C₃N₂(Y^{j})₄⁺, les ions imidazolinium C₃N₂(Y^{j})₇⁺, les ions triazolium C₂N₃(Y^{j})₄⁺, le nitrosyle et NO₂⁺, les ions sulfonium [S(Y^{j})₃]⁺, les ions phosphonium [P(Y^{j})₄]⁺ et les ions iodonium [I(Y^{j})₂]⁺. Dans les différents ions onium précités, les substituants Yⁱ d'un même anion peuvent être identiques ou différents. Ils représentent indépendamment les uns des autres H ou l'un des substituants indiqués ci-dessus pour Y.

Lorsque M^{m+} est un cation organo-métallique, il peut être choisi parmi les ions métallocéniums. A titre d'exemple, on peut citer les cations dérivés du ferrocène, du titanocène, du zirconocène, d'un indénocénium ou d'un arène métallocénium. Il peut également être choisi parmi les cations métalliques coordonnés par des atomes tels que O, S, Se N, P ou As, portés par des molécules organiques, notamment sous forme de ligands carbonyle, phosphine ou porphyrine possédant éventuellement une chiralité. M^{m+} peut également être choisi parmi les groupes trialkyl-silyle, tétraalkyl-germanyle ou dialkyl-stannyle dans lesquels les radicaux alkyle ont de 1 à 10 atomes de carbone ; dans ce cas, M est relié au groupement [R¹-X¹(Z¹)-Q-X²(Z²)-R²] par une liaison covalente très labile et le composé se comporte comme un sel.

Les composés avec R_{F}SO₂N et ceux dans lesquels R¹ représentent un groupe perfluoré ou un groupement alkyle sont particulièrement intéressants, notamment pour la forte acidité et la dissociation des sels correspondants.

Dans les composés selon l'invention, les radicaux R1 et R2 sont de préférence choisis indépendamment l'un de l'autre parmi les radicaux perfluoroalkyles ayant de préférence de 1 à 6 atomes de carbone, les radicaux alkyles ayant de préférence de 1 à 8 atome de carbone, les radicaux alkényle ayant de préférence de 2 à 18 atomes de carbone, les radicaux dialkylamino dans lesquels les radicaux alkyles ont de préférence de 1 à 18 atomes de carbone, et les radicaux styrényles.

Le choix des substituants Rⁱ dans les composés de la présente invention permet d'obtenir des composés dans lesquels l'anion possède une chiralité intrinsèque sur un atome de soufre. De tels composés sont utiles pour induire une sélectivité éniantomérique lors de la préparation de composés organiques actifs ou pour induire une stéréosélectivité dans les réactions de polymérisation. Parmi ces composés, on peut citer ceux qui répondent à la formule suivante [R¹SO₂N-S*=O(R²)=NSO₂R⁶]⁻ dans laquelle R¹ est différent de R⁶. On préfère tout particulièrement les composés dans lesquels R¹ et R⁶ représentent indépendamment l'un de l'autre un radical choisi parmi F, CF₃, C₂F₅, C₄F₉, C₆F₁₃ et C₈F₁₇, et R² et R⁵ représentent indépendamment l'un de l'autre un alkyle, un aryle, un alkylaryle ou un dialkylamino ayant de préférence de 1 à 20 atomes de carbone.

Les composés ioniques de la présente invention peuvent être préparés par divers procédés.

De manière générale, un composé répondant à la formule 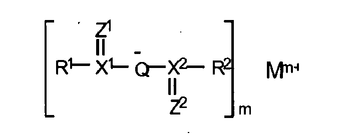 est préparé en faisant réagir un composé précurseur noté ci-après (Z¹,L) comprenant le groupe Z¹ et un groupe partant L sur un dérivé A²Z² du groupe Z² selon l'un des schémas réactionnels suivants :

R¹-X¹(Z¹)-Q-X²(L)R² + A₂Z² ⇒ [R¹-X¹(Z¹)-X²(L) (Z²)R²]⁻A⁺ + LA

ou

R¹-X¹(Z¹)-QA₂ + L-X²(Z²)R² ⇒ [R¹-X¹(Z¹)-X²(L)(Z²)R²] +A⁻ + LA

A représente un métal alcalin, un proton, une base aminée ou phosphorée, un groupe trialkyl silyle, un groupe dialkyle stannyle, MgL1, ZnL1, CdL1, Cu, Mg, Zn, Cd, Hg, un groupement trialkyl-silyle, trialkyle-germanyle ou trialkyle-stannyle.

Les groupes partants L ou L1 sont avantageusement choisis parmi les halogènes, les pseudohalogènes incluant les sulfonates fluorés ou non, les radicaux imidazoyle, triazolyle et benzotriazoyle.

Les composés (Z¹,L) dans lesquels le groupe partant est un halogène peuvent être préparés par exemple par action d'un agent halogénant sur un sel R¹-X¹(Z¹)-Q-X²(O)(R²)⁻A⁺ ou sur l'acide correspondant. Le cation A est de préférence choisi parmi les cations de métal alcalin, les ions ammoniums inorganique NH₄⁺ ou organiques R³NH⁺ (y compris le pyridinium) et l'ion Ag⁺ qui présente une forte affinité pour Cl, Br, I.

Parmi les agents halogénants utiles, on peut citer SF₄, le trifluoro(diéthylamino)soufre IV (DAST), le chlorure de thionyle, le chlorure d'oxalyle, le fluorure d'oxalyle, le pentachlorure de phosphore, le mélange PΦ₃ + CCl₄, le chlorure de (chlorométhylène)diméthyl ammonium [CH(Cl)=N(CH₃)₂]⁺Cl⁻ ou son homologue dérivé de la N-méthylpyrrolidinone, l'iodure de 1-méthyl-2-fluoro pyridinium. La préparation d'un composé (Z¹,L) dans lequel le groupe partant est un halogène est illustrée schématiquement par l'exemple suivant :

[CF₃SO₂NSO₂(C₄H₉)]Na + (COCl) ⇒ CO + CO₂ + [CF₃SO₂NSO(C₄H₉)Cl] + NaCl

Les composés précurseurs (Z¹,L) dans lesquels le groupe partant est l'imidazolyle, le triazolyle ou le benzotriazolyle, peuvent être obtenus par action de leur sel alcalin, de leur dérivé du triméthyl-silyle ou de leur dérivé diméthyl-stannyle sur le composé précurseur halogéné correspondant, par exemple selon le schéma réactionnel suivant : [CF₃SO₂NSO(C₄H₉)Cl] + ImSi(CH₃)₃ ⇒ ClSi(CH₃)₃ + [CF₃SO₂NSO(C₄H₉)Im], dans lequel Im représente

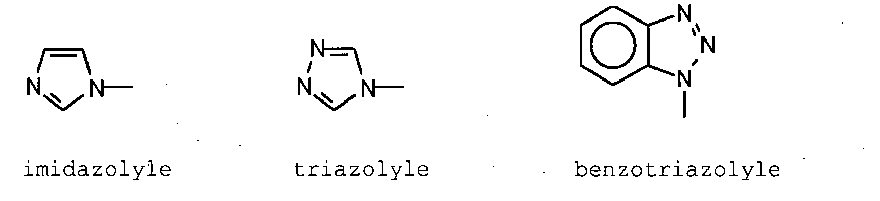

Les composés précurseurs (Z¹,L) dans lesquels le groupe partant est un pseudo-halogène tel qu'un sulfonate peuvent être obtenus par action du chlorure d'acide ou de l'anhydride de l'acide sulfonique correspondant au sulfonate, sur un sel précité R¹-X¹(Z¹)-Q-X²(O)R²⁻M⁺. La réaction d'un sel d'argent sur un chlorure de l'acide sulfonique R⁷SO²Cl (R⁷ étant de même nature que les Rⁱ) selon le schéma suivant est particulièrement avantageuse :

R¹-X(Z¹)-Q-X(O)R²⁻Ag⁺ + R⁷SO₂Cl ⇒ R¹-X(Z¹)-Q-X(SO₃R⁷)R² + AgCl

De manière, générale, il est avantageux de préparer les composés précurseurs R¹-X¹(Z¹)-Q-X²(L)R² à partir d'un composé comprenant un anion dans lequel le soufre ou le phosphore sont à l'état d'oxydation respectivement IV et III. Par oxydation de ces anions, les dérivés de soufre VI ou du phosphore V sont obtenus selon le schéma réactionnel

R¹-X(Z¹)-Q-X²(R²)⁻A⁺ + 2L ⇒ R¹-X(Z¹)-Q-X²(L)R² + LA

Les composés préférés pour L sont les halogènes, tels le fluor, le chlore, le brome.

Les différents groupes partants peuvent être échangés par des techniques bien connues de l'homme de métier. Par exemple, le chlore peut être remplacé par le fluor par action d'un agent possédant des ions fluorure actifs et une affinité pour les ions chlore, tel que le fluorure d'argent AgF ou le fluorure de tétraméthyl ammonium, le fluorure de 1,1,1,3,3,3-hexakis(diméthylamino)diphosphazénium {[(CH₃)₂N]₃P}₂N⁺F⁻ ou, le fluorure de tétrakis(tris(diméthylamino)phosphoranylidèneaminophosphonium {[(CH₃)₂N]₃P=N}₄P⁺F⁻, le composé d'addition du fluorure de tris-(diméthylamino)sulfonium avec le triméthylfluorosilane [(CH₃)₂N]₃S⁺[Si(CH₃)₃F₂]⁻.

Les dérivés de l'imidazole ou du triazole peuvent être obtenus par action de leur sel alcalin ou de leur dérivé du triméthyl-silyle ou du diméthyl-stannyle) sur le dérivé correpondant, selon le schéma réactionnel :

[CF₃SO₂NSO(C₄H₉)Cl] + ImSi(CH₃)₃ ⇒ ClSi(CH₃)₃ + [CF₃SO₂NSO(C₄H₉)Im]

dans lequel Im représente l'imidazolyle, le triazolyle ou le benzotriazolyle.

Un composé symétrique, dans lequel X¹(Z¹) est identique à X²(Z²) peut être préparé par action d'un nitrure ionique ou d'un dérivé métallique d'hexaméthydisilazane ou d'ammoniac en présence d'une base sur un précurseur possédant un groupe partant L, selon le schéma réactionnel suivant, dans lequel R, X et Z ont la signification donnée ci-dessus respectivement pour Rⁱ, Xⁱ et Zⁱ, A et L sont tels que définis ci-dessus : 2RX(Z)L + A₃N ⇒ [RX(Z)]₂N⁻A⁺ + 2LA. Par exemple :

2CF₃SO(=NSO₂CF₃)F + Li₃N ⇒ [CF₃SO(=NSO₂CF₃)]₂N⁻A⁺ + 2LiF

L'agent nitrurant peut être avantageusement Li₃N, l'ammoniac, ses dérivés avec les silanes et leurs dérivés alcalins tels N[SiCH₃)₃]₂Li, N[SiCH₃)₃]₂Na et N[SiCH₃)₃]₂K.

Un composé de formule [R¹SO₂N-S*=O(R²)=NSO₂R⁶]⁻ M⁺ (VIId) peut être obtenu en faisant réagir un sel [R¹SO₂NSO₂R²]⁻ M'⁺ avec un agent halogénant, pour obtenir le précurseur R¹SO₂NSOR²(X) (X étant un halogène). Ledit précurseur est ensuite condensé sur une sulfonamide R⁶SO₂NH₂ en présence d'une base ou sur ses dérivés métalliques comme R⁶SO₂NLi₂ ou R⁶SO₂NNa₂. Le cation souhaité pour le composé final est obtenu par des procédés classiques d'échange d'ions.

De la même manière, les carbures ioniques permettent de préparer les composés [RX(Z)]₃C⁻A⁺, selon le schéma réactionnel 2RX(Z)L + A₄C ⇒ [RX(Z)]₃C⁻A⁺ + 3LA.

Les composés de la présente invention sont utilisés pour la catalyse de divers types de réactions chimiques, et notamment pour des réactions de polymérisation, des réactions de condensation, des réactions d'addition ou d'élimination, des réactions d'oxydation ou de réduction, des solvolyses, des réactions de Friedel-Crafts et des réactions de Diels-Alder. Pour ces applications en catalyse, les composés seront choisis essentiellement en fonction du cation associé à la partie anionique.

Pour la catalyse de réactions de Diels Alder ou de réactions de Friedel-Crafts, les cations de métal alcalin, de métal alcalino-terreux, de métal de transition ou de terre rare conviennent. Les composés ayant un cation H⁺, Li⁺, Mg⁺⁺, Ca⁺⁺, Cu⁺⁺, Zn⁺⁺, Al⁺⁺⁺, Fe⁺⁺⁺ ou Fe⁺⁺⁺ sont préférés.

Les composés de l'invention dans lesquels le cation est un onium du type diazonium, sulfonium, iodonium, métallocénium, peuvent être utilisés comme amorceurs de polymérisation cationique, notamment pour polymériser ou réticuler les éthers vinyliques, les époxydes, les acétals et éthers cycliques, les vinylamides, les oxazolines, l'isobutylène, le styrène ou les siloxanes. Sous l'action d'un rayonnement actinique, de tels composés génèrent la forme acide correspondante capable d'amorcer une réaction de polymérisation cationique. Un composé de l'invention peut être utilisé en tant que photoamorceur éventuellement en présence d'un sensibilisateur, ou d'un amorceur radicalaire amorçable thermiquement ou par un rayonnement actinique. Les composés de l'invention sous forme de sel· d'amine peuvent servir d'amorceur des polymérisations cationiques par chauffage libérant la forme protonique correspondante. De même, si le cation est un sel d'un composé azoïque cationique (par exemple tel que représenté ci-dessous) il peut servir par chauffage d'amorceur des polymérisations radicalaires.

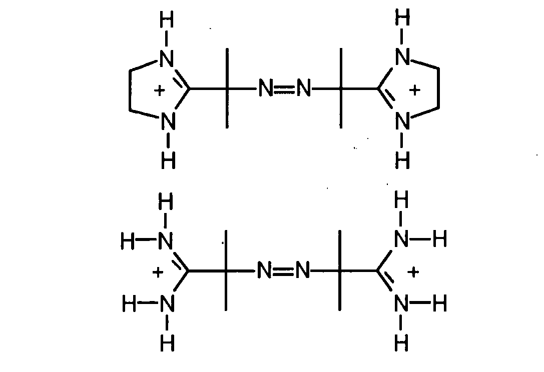

La présente invention permet d'obtenir des composés dans lesquels l'anion présente une chiralité intrinsèque, ce qui permet d'induire des asymétries énantiomériques lors de l'utilisation desdits composés en tant que catalyseurs, de préparer des polymères stéréoréguliers et de conférer un pouvoir rotatoire aux matériaux les contenant.

La présente invention est expliquée plus en détails par les exemples suivants, qui décrivent la préparation et diverses utilisations de composés de l'invention. L'invention n'est toutefois pas limitée à ces exemples.

### Exemple 1

Le composé du soufre à l'état IV [CF₃SO₂NS(O)CF₃]⁻Na⁺ a été préparé selon l'un ou l'autre des deux schémas réactionnels possibles :

CF₃S-SCF₃ + CF₃SO₂NCl₂ ⇒ CF₃SO₂NS(Cl)CF₃ + CF₃SCl CF₃SO₂NS(Cl)CF₃ + NaOS(CH₃)₃ ⇒ [CF₃SO₂NS(O)CF₃]⁻Na⁺ ClSi(CH₃)₃ a)

ou

CF₃SO₂NNa₂ + CF₃SO₂Cl ⇒ [CF₃SO₂NS(O)CF₃]⁻Na⁺ + NaCl b)

Le composé halogéné CF₃SO₂NS(Cl)CF₃ a été préparé par chloruration du sel de sodium [obtenu par l'une des voies a) ou b)] en l'absence de solvant et transformé en dérivé fluoré par action de N(CH₃)₄F dans l'éther à -35°C.

2,67 g du composé halogéné CF₃SO₂NS(F)CF₃ dans 20 ml de THF ont été mis à réagir avec 170 mg de nitrure de lithium et l'on a obtenu le sel :

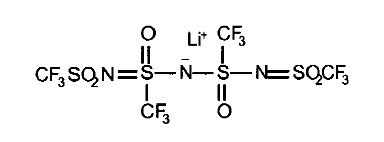

### Exemple 2

15,6 de chlorure de butane sulfonyle ont été dissous dans 100 ml d'acétonitrile anhydre auquel ont été ajoutés 14,9 g de trifluorméthane sulfonamide et 20,4 g de 1,4-diazabicyclo-2,2,2-octane (DABCO). Le mélange a été agité pendant 4 heures à température ambiante, puis le chlorhydrate de DABCO formé a été éliminé par centrifugation et le solvant est évaporé. Le résidu solide a été repris par 100 ml d'une solution saturée de KCl dans l'eau à laquelle on a ajouté 15 ml d'acide acétique. Le précipité de [C₄H₉SO₂NSO₂CF₃]⁻K⁺ a été filtré et purifié par cristallisation dans l'eau chaude.

9,22 g de sel précédemment obtenu ont été mis en solution dans 50 ml d'acétonitrile anhydre auxquels on a ajouté 2,6 ml de chlorure d'oxalyle (COCl)₂ et trois gouttes de DMF servant de catalyseur. Après cessation du dégagement gazeux, on a ajouté 4,4 g de trifluorométhane sulfonamide et 6,73 g de DABCO. Après agitation à température ordinaire, le chlorhydrate de DABCO formé a été éliminé par centrifugation et la solution a été versée dans 100 ml d'eau contenant 15% en poids de KCl et 15 ml d'acide acétique. Le précipité a été séparé, lavé à l'eau et recristallisé dans l'éthanol. I1 répond à la formule :

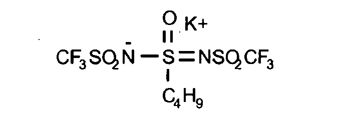

Le schéma réactionnel des étapes successives du procédé est comme suit

[C₄H₉SO₂NSO₂CF₃]⁻K⁺ + (COCl)₂ ⇒ KCl + C₄₀H₉SO(Cl)NSO₂CF₃

C₄H₉SO(Cl)NSO₂CF₃ + 2N(C₂H₄)₃N ⇒ N(C₂H₄)₃NHCl + [C₄H₉SO(NSO₂CF₃)NSO₂CF₃]-N(C₂H₄)₃NH⁺

[C₄H₉SO(NSO₂CF₃)NSO₂CF₃]⁻N(C₂H₄)₃NH⁺ + KCl ⇒ N(C₂H₄)₃NHCl + [C₄H₉SO(NSO₂CF₃)NSO₂CF₃]⁻K⁺,

### Exemple 3

Le composé [C₈H₁₇SO₂NSO₂CF₃]⁻K⁺ a été préparé par une procédure similaire à celle de l'exemple 2 en utilisant le chlorure d'octane sulfonyle. 36,3 g de ce composé ont été mis en solution dans la DMF anhydre et on a ajouté 13 g de chlorure de (chlorométhylène)diméthyl ammonium [CH(Cl)=N(CH₃)₂]⁺Cl⁻. Un précipité de KCl s'est formé et a été éliminé par filtration. A la solution, on a ajouté 1,7 g de nitrure de lithium. Après 24 heures d'agitation à température ordinaire, le produit de la réaction a été centrifugé et le surnageant a été versé dans 200 ml d'eau saturée en chlorure de potassium. Le précipité pâteux a été décanté et lavé plusieurs fois à l'eau, puis extrait par 50 ml d'un mélange équivolumique diéthoxy-2-éthane et de dichlorométhane. Après évaporation du solvant, on a obtenu un sel hydrophobe ayant pour formule :

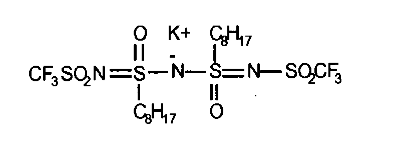

### Exemple 4

253 g de polyaniline protonée sous forme de chlorure ont été mis en suspension dans l'acétonitrile et on a ajouté 6,7 g du composé de l'exemple 3. Le mélange a été agité pendant 24 heures et le polymère, dans lequel les ions chlorures ont été échangés par l'ion (CF₃SO₂NSOC₈H₁₇)₂N⁻, a été lavé à l'eau et à l'éthanol pour éliminer le KCl, puis séché. Le polymère conjugué sous forme dopée conductrice est soluble dans les solvants de faible polarité comme les xylènes, le dichloroéthane ou le chloroforme. La conductivité du polymère est supérieure à 1 Scm⁻¹ et stable vis-à-vis des agents atmosphériques, en particulier l'humidité.

### Exemple 5

Le composé ionique [CF₃SO₂NSO₂(3,5-C₆H₃(CF₃)₂]⁻K⁺ a été préparé par une méthode similaire à celle de l'exemple 2 à partir de chlorure de 3,5-bis(trifluorométhyl)benzène sulfonyle et de trifluométhane sulfonamide. 18,6 g de sel ont été traités par 7 g de DAST (C₂H₅)₂NSF₃. Le composé fluoré obtenu : 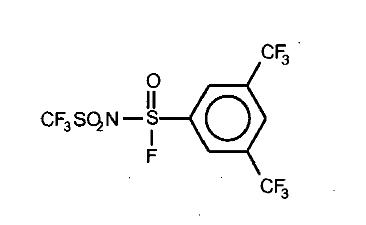 a été purifié par distillation sous vide. 4,27 g de ce composé ont été ajoutés à 30 ml de THF anhydre contenant 670 mg de malononitrile et 18 mg d'hydrure de lithium. A la fin de la réaction, observée par la fin du dégagement d'hydrogène, le mélange réactionnel a été filtré et le THF évaporé. Le résidu solide a été repris par l'eau et filtré. On a ajouté 4,4 g de sulfate de brucine dans 50 ml d'eau et le mélange réactionnel a été agité pendant 24 heures. Après séparation et séchage, 8 g du précipité formé ont été traités par une solution de 1,6 g d'une solution de tétraphénylborate de sodium dans 20 ml d'une solution équivolumique eau-éthanol. La solution après filtration a été séchée pour donner le sel de sodium de l'anion intrinsèquement chiral par son atome de soufre, résolu en isomère actif par la brucine :

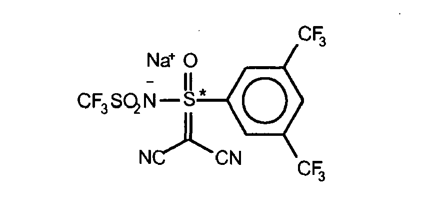

Les sels de lithium, de magnésium ou de terres rares et d'yttrium de cet anion induisent des excès énantiomériques de 50 à 92% lors de la catalyse des réactions de Diels Alder et des condensations aldoliques. Un catalyseur de polymérisation cationique a été préparé par action du chlorure d'acétyle en quantité stoechiométrique sur le sel d'argent, lui-même obtenu par échange du sel de sodium par le toluène-sulfonate d'argent dans un mélange isopropanol-toluène. Ce catalyseur induit une polymérisation de l'oxyde de propylène en un polymère optiquement actif. D'une façon similaire, l'éther méthyl-vinylique est polymérisé en une macromolécule isotactique cristalline insoluble dans l'eau, contrairement aux polymères obtenus avec les amorceurs cationiques non chiraux. Les composés : 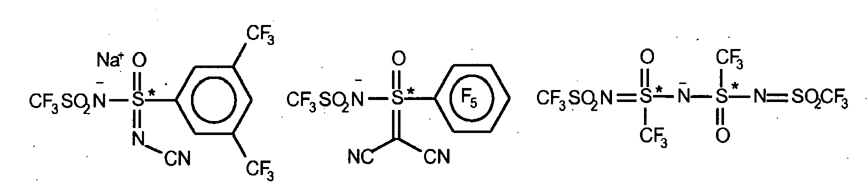 présentent aussi une chiralité intrinsèque sur le centre anionique, permettant la catalyse de réactions favorisant un éniantomère, les polymérisations donnant des polymères optiquement actifs ou présentant une tacticité.

### Exemple 6

Le composé (C₄H₉SO₂)₂N]Na a été préparé selon la méthode de Runge et al. (Chem. Ber. 88-4, 533 (1955)) et halogéné par le chlorure de thionyle dans l'acétonitrile, la réaction étant catalysée par le DMF. Le chlorure C₄H₉SO₂NSO(Cl)C₄H₉ en solution dans le THF a été traité par l'ammoniac de manière à former la sulfimidosulfamide C₄H₉SO₂NSO(NH₂)C₄H₉. On a fait réagir des quantités équimoléculaires du chlorure et de l'amide dans la pyridine pour former le sel de pyridinium :

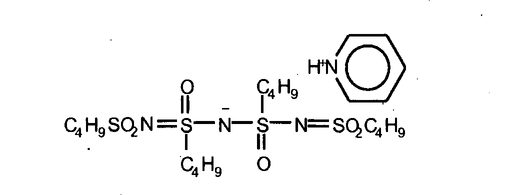

Le sel du colorant rhodamine 6G de cet anion a été précipité par simple mélange dans l'eau du perchlorate de rhodamine 6G et du sel de pyridinium. Ce sel possède une solubilité marquée dans un grand nombre de composés organiques, en particulier dans les monomères de type méthacrylate de méthyle ou styrène, et cette solubilité est maintenue lors de la polymérisation de ces monomères. Les solutions solides ainsi constituées sont hautement fluorescentes et permettent la réalisation de lasers solides, en films minces ou en fibres.

### Exemple 7

Le dichlorure de bis (indènyle)zirconium a été traité par le sel d'argent du composé de l'exemple 4 et l'on a obtenu le composé 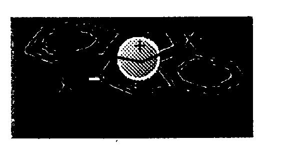 dans lequel X⁻ est [CF₃SO₂NSOC₈H₁₇]₂N⁻.

Ce métallocène possède une excellente solubilité dans les solvants usuels de polymérisation tels que le toluène ou les hydrocarbures aliphatiques, et il montre une activité notable pour la polymérisation des α-oléfines, notamment pour l'éthylène et le propylène.

### Exemple 8

A une solution de 2,43 g (8,25 mmoles) de fluorure de N-(trifluorométhylsulphonyl)phénylsulfonimidoyle préparé suivant la méthode décrite par Garlyauskajte & ali (Tetrahedron, vol. 50, p. 6891, 1994), dans 4 ml de THF anhydre, on a ajouté sous argon 0,14 g (4,12 mmoles) de Li₃N et 0,04 g (0,33 mmole) de 4-diméthylaminopyridine comme catalyseur. Le milieu réactionnel a ensuite été chauffé au reflux durant 24 heures. Après refroidissement et évaporation du solvant sous vide, le résidu obtenu a été dissous dans 10 ml d'eau, la solution filtrée, puis passée sur une colonne échangeuse d'ions Amberlite IR-120 (forme acide). A cette solution, on a ajouté une solution à 50% d'hydroxyde de potassium. Le précipité formé a été séparé, recristallisé dans l'eau puis séché par une distillation azéotropique avec du benzène. On a ainsi obtenu le composé suivant :

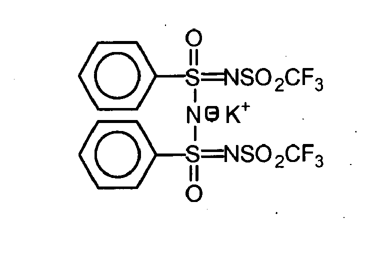

On a obtenu le sel de lithium correspondant par échange ionique avec du chlorure de lithium dans le THF. Les solutions concentrées de ce sel de lithium dans l'éther sont actives pour la catalyse des réactions de Diels & Alder.

### Exemple 9

A une solution dans 4 ml de THF anhydre de 174 mg de Li₃N (10 mmoles), on a ajouté 2,83 g (10 mmoles) de fluorure de N-(trifluorométhylsulfonyl)trifluorométhylsulfonimidoyle, préparé suivant la méthode décrite dans l'exemple 1, en solution dans 4 ml de THF anhydre, puis 0,04 g (0,33 mmole) de 4-diméthylaminopyridine comme catalyseur. Le milieu réactionnel a ensuite été agité durant 24 heures. Après évaporation des solvants sous vide, le résidu obtenu a été repris dans une solution saturée de KCl. Le précipité formé a été séparé, recristallisé dans l'eau, puis séché par une distillation azéotropique avec du benzène. On a ainsi obtenu le composé suivant :

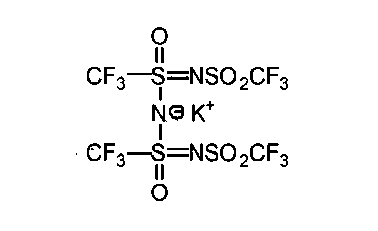

Suivant le même procédé, on a obtenu à partir de [C₄F₉SO₂NSOC₄F₉]⁻Na⁺ le composé suivant :

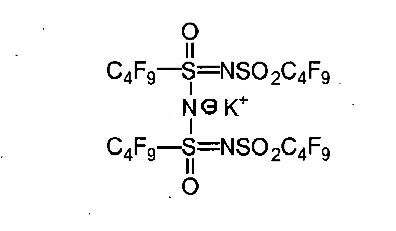

### Exemple 10

A une solution à 0°C et sous argon de 14,36 g (100 mmoles) de chlorure de sul-famoyle (CH₃)₂NSO₂Cl (commercialisé par Aldrich) et de 14,91 g de trifluorométhanesulfonamide CF₃SO₂NH₂ (100 mmoles), préparés selon le mode opératoire de l'exemple 2, dans 60 ml de tétrahydrofurane anhydre, on a ajouté 22,44 g (200 mmoles) de 1,4-diazabicyclo[2.2.2]octane (DABCO) en solution dans 20 ml de tétrahydrofurane anhydre à 0°C. Après 2 heures à 0°C, la réaction a été poursuivie pendant 24 heures à l'ambiante. Le précipité d'hydrochlorure de DABCO a été éliminé par filtration sur un verre fritté de porosité N°4. Après évaporation du tétrahydrofurane et séchage, le produit a été solubilisé dans 25 ml d'éthanol. On a alors ajouté 9,81 g (100 mmoles) d'acétate de potassium CH₃COOK, puis on a recristallisé le précipité obtenu au reflux de l'éthanol. Après refroidissement, filtration et séchage, on a récupéré le sel de potassium (CH₃)₂NSO₂NKSO₂CF₃. 50 mmoles de ce sel ont été mis en solution dans 30 ml de THF, puis traités par 50 mmoles de chlorure d'oxalyle. Il s'est formé rapidement un précipité de chlorure de potassium qui a été éliminé par filtration. A la solution de (CH₃)₂NS(Cl)O=NSO₂CF₃, on a alors ajouté, sous argon, 5 mmoles de Li₃N. Après 72 heures sous agitation, le solvant a été évaporé et le résidu recristallisé dans une solution saturée de chlorure de potassium. On a obtenu le composé suivant :

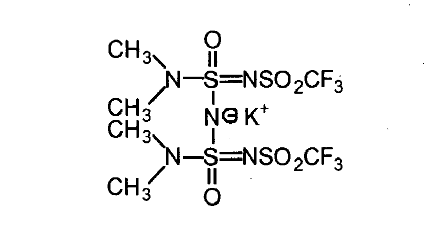

Suivant le même procédé, on a obtenu, en remplaçant le chlorure de sulfamoyle par le chlorure de butanesulfonyle, le composé suivant :

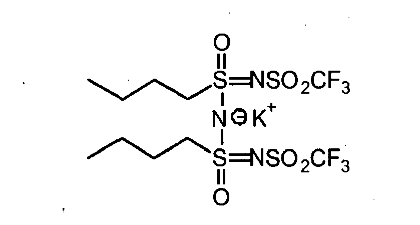

### Exemple 11

A 10 mmoles de fluorure de N-(trifluorométhylsulphonyl)trifluorométhylsulfonimidoyle préparé suivant la méthode décrite dans l'exemple 1, en solution dans 10 ml de pyridine anhydre, on a ajouté, sous argon, 10 mmoles de p-styrènesulfonamide, 0,04 g (0,33 mmole) de 4-diméthylaminopyridine comme catalyseur, puis 0,1 mmole de tertiobutylhydroquinone. Le milieu réactionnel a ensuite été agité durant 24 heures à 40°C. Après évaporation de la pyridine sous vide, le résidu obtenu a été repris dans le THF puis agité 24 heures en présence d'un excès de phosphate de potassium K₃PO₄. Après filtration et évaporation du solvant, on a obtenu le composé suivant :

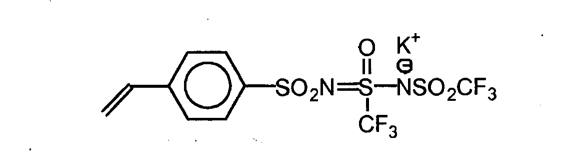

### Exemple 12

Suivant un procédé similaire à celui de l'exemple 11, et en remplaçant le p-styrènesulfonamide par de l'allylsulfonamide, on a obtenu le composé suivant :

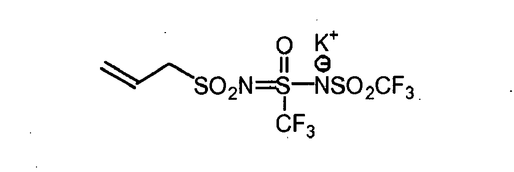

### Exemple 13

5 mmoles de chlorure de diphényliodonium (C₆H₅)₂ICl et 5 mmoles du sel de potassium [C₄H₉SO₂N=S(=O)(CF₃)]₂NK décrit à l'exemple 10 ont été agités ensemble durant 24 heures dans l'eau. Par extraction de la phase aqueuse par du dichlorométhane, on a récupéré après évaporation du dichlorométhane et séchage, le composé suivant :

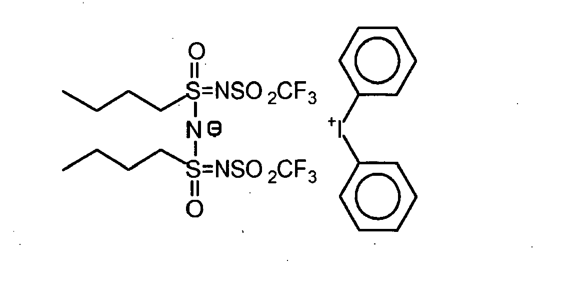

Ce sel permet d'amorcer sous l'effet d'un rayonnement actinique (lumière, rayons γ, faisceaux d'électrons) les réactions de polymérisation ou les réactions de réticulation cationique de monomères riches en électrons, en particulier les éthers vinyliques, les propényléthers, les époxydes, l'isobutylène ou la N-vinylpyrolidinone.

Il est soluble dans la plupart des solvants organiques usuels (tétrahydrofurane, acétonitrile, diméthylformamide, acétate d'éthyle, glymes, toluène, ...) et dans les polymères solvatants aprotiques comme le polyoxyde d'éthylène. Il est aussi soluble à plus de 10% en poids dans les solvants réactifs comme le triéthylèneglycol divinyl éther, ou le cyclohexane diméthanol divinyl éther, contrairement par exemple au sel de bis(trifluorométhanesulfonyl)imide du diphényliodonium.

On a testé les propriétés de photo-amorçage de ce sel en irradiant par un rayonnement U.V. à 254 nm, d'une puissance de 1900 mW/cm², une solution de triéthylèneglycol divinyl éther le contenant à 1% en poids. Après quelques secondes sous irradiation, le solvant réactif a pris en masse, cette réaction étant très exothermique.

### Exemple 14

L'allylsulfonimidure de l'exemple 12 a été époxydé par le sel de magnésium de l'acide peroxypthalique commercial et l'on a obtenu le sel :

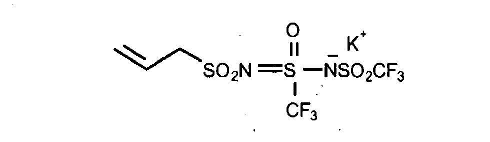

Dans un réacteur chimique de type Parr®, on a introduit une solution dans 100 ml de tétrahydrofurane anhydre de 50 mmoles dudit sel, et de 6 mmoles d'allylglycidyléther. Après avoir purgé le réacteur avec de l'argon, on a introduit à l'aide d'une vanne, 146 mmoles d'oxyde d'éthylène, puis 100 µl d'une solution 10⁻² M de *t*-butoxyde de potassium dans le THF. On a alors effectué sous argon la polymérisation en chauffant le milieu réactionnel à 60°C pendant 48 heures. Après refroidissement, la solution a été concentrée, puis le polymère a été récupéré par reprécipitation dans l'éther. Après filtration et séchage, les cations potassium de ce polyélectrolyte ont été échangés par des cations lithium par passage sur une colonne échangeuse de cations. On a ainsi obtenu le polyélectrolyte suivant : 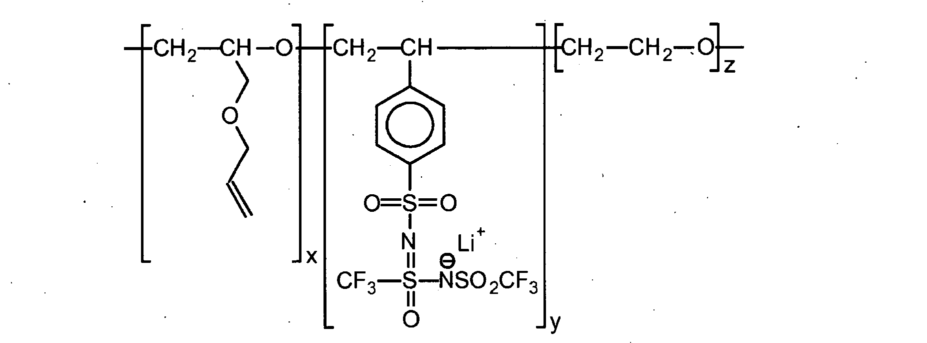 x:y:z étant tel que 6:50:200 du fait de la plus grande réactivité de l'oxyde d'éthylène. Ce polymère permet de réaliser des électrolytes polymères ou des électrolytes gélifiés à anions fixés, le polymère assurant la double fonction de matrice permettant d'obtenir le gel et de polyélectrolyte.

On a ainsi réalisé un électrolyte gélifié de 40 µm d'épaisseur contenant (en poids) 30% du polyélectrolyte ci-dessus, 35% de carbonate d'éthylène et 35% de carbonate de propylène, après réticulation des fonctions allyles par irradiation UV en présence de 1,2-diphényl-1-kéto-2,2-diméthoxyéthane. Ce gel présente de bonnes propriétés mécaniques et une conductivité supérieure à 10⁻³ S.cm⁻¹ à 30°C. Le nombre de transport cationique dans cet électrolyte a été estimé à 0,85.

On a assemblé un générateur électrochimique en utilisant comme électrolyte, l'électrolyte gélifié décrit ci-dessus. Le matériau d'anode était un coke de carbone (80% en volume) mélangé avec le copolymère (PANSDTFSI) de cet exemple sous la forme non réticulée comme liant (20% en volume). Le matériau de la cathode était un matériau composite constitué par du noir de carbone (6% en volume), LiCoNiO₂ (75% en volume) et du copolymère non réticulié (PANSDTFSI) (20% en volume). Ce générateur a donné de bonnes performances en cyclage à 25°C. On a pu réaliser 1000 cycles de charge/décharge entre 3 et 4,2 V en conservant une capacité supérieure à 80% de la capacité au premier cycle. Le générateur présente de très bonnes performances lors d'un appel de puissance du fait de l'utilisation d'anions fixés. L'utilisation d'anions fixés a également permis d'améliorer l'évolution de la résistance d'interface.

Cette famille de polymères est d'un grand intérêt pratique pour le développement de générateurs électrochimiques.

### Exemple 15

Par échange ionique dans l'acétone entre le sel de potassium [C₄H₉SO₂N=S(=O)(CF₃)]₂NK décrit à l'exemple 10 avec l'iodure de 3,3'-diéthylthiatricarbocyanine (qui est un colorant infrarouge de la famille des cyanines, commercialisé par Aldrich), suivi d'une reprécipitation dans l'eau, on a obtenu après filtration et séchage le composé suivant :

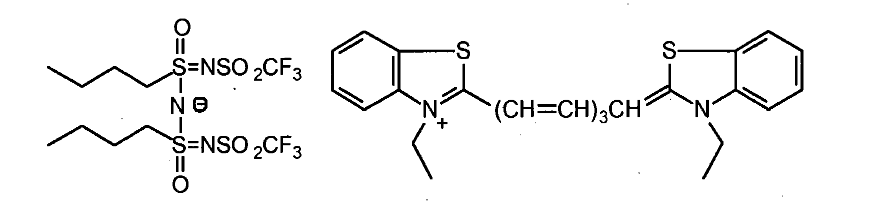

Ce sel est très soluble dans les solvants peu polaires comme le dichlorométhane ou le chlorure de méthylène, ainsi que dans les matrices polymères peu polaires comme le polyméthacrylate de méthyle.

D'autre part, on a constaté une très nette diminution de l'agrégation des colorants cationiques entre eux du fait du caractère "plastifiant" des groupements di-2-éthylhexylamino. Cette diminution de l'agrégation est un avantage dans la mesure où le phénomène d'agrégation amène un élargissement des bandes d'absorption optiques préjudiciable à la précision de fonctionnement des systèmes utilisant des colorants, en particulier les disques optiques de stockage d'informations.

### Exemple 16

Dans un ballon tricol équipé d'un réfrigérant, d'une agitation mécanique et d'une entrée de gaz neutre (Argon), 100 mmoles de triméthoxysilane ont été mis en solution dans du tétrahydrofurane ; on a alors ajouté 100 mmoles du sel de potassium décrit à l'exemple 12 et 70 mg d'acide chloro-platinique H₂PtCl₆. Le mélange a été chauffé au reflux pendant 4 heures. Après refroidissement et évaporation du THF, on a obtenu le composé suivant :

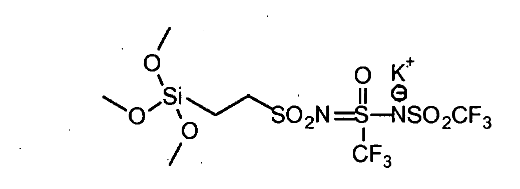

Ce composé a été greffé à la surface de particules de silice préalablement traitées dans une solution d'acide chlorhydrique. De telles particules de silice sont particulièment utiles pour effectuer de la catalyse supportée en utilisant un cation approprié.

### Exemple 17

10 mmoles du sel de potassium [C₄F₉SO₂N=S(=O)(CF₃)]₂NK, décrit à l'exemple 9, ont été traités par du tétrafluoroborate d'argent dans un mélange 80/20 de toluène/ dioxane. Après quelques heures sous agitation, le milieu réactionnel a été filtré pour éliminer le précipité de tétrafluoroborate de potassium, puis on a fait buller du chlorure d'hydrogène anhydre gazeux dans le toluène. Après filtration pour éliminer le chlorure d'argent formé, et addition de particules de silice, le solvant a été évaporé, et on a obtenu l'acide [C₄F₉SO₂N=S(=O)(CF₃)]₂NH déposé sur la silice. Dans un réacteur Parr, on a introduit 1 moles d'octane et 10 mmoles d'acide supportés par la silice, puis on a porté le réacteur à 200°C pendant 10 mn. On a ainsi isomérisé l'octane en isooctane.

L'acide [C₄F₉SO₂N=S(=O)(CF₃)]₂NH déposé sur la silice et utilisé comme catalyseur d'isomérisation présente un excellent taux d'utilisation et il est facile à récupérer du fait de sa faible volatilité et de son caractère oléophobe, c'est-à-dire de son insolubilité dans les hydrocarbures aliphatiques.

L'acide [C₄F₉SO₂N=S(=O)(CF₃)]₂NH en solution dans des solvants fluorés comme le Fluorinert® (commercialisé par la société 3M) peut également être utilisé pour effectuer des réactions chimiques mettant en jeu une catalyse acide en milieu biphasique, les produits de réaction, insolubles dans le fluide fluoré, étant récupérés par simple décantation.

## Revendications

1. Utilisation d'un composé ionique en tant que catalyseur pour une réaction de polymérisation, une réaction de condensation, une réaction d'addition ou d'élimination, une réaction d'oxydation ou de réduction, une solvolyse, une réaction de Friedel et Craft ou une réaction de Diels Alder, **caractérisée en ce que** ledit composé ionique répond à **l'une des** formule**s suivantes :** 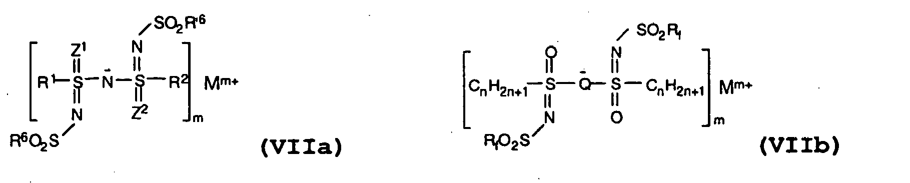 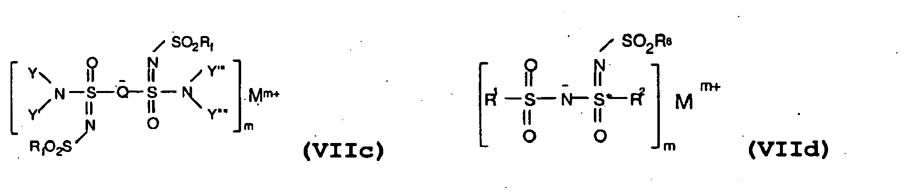 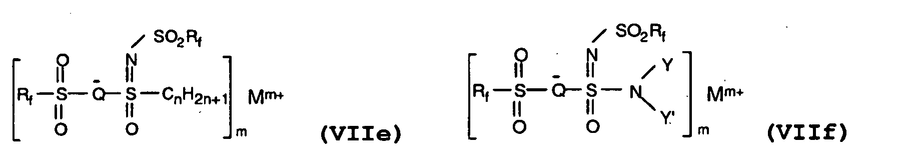 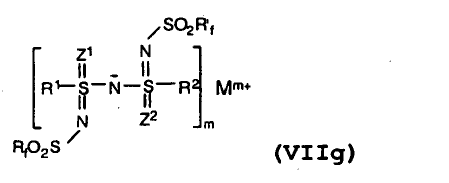 dans **lesquelles :**
- M^{m+} est le proton, ou un cation métallique ayant la valence m choisi parmi les ions des métaux alcalins, des métaux alcalino-terreux, des métaux de transition, des terres rares, ou un cation organique onium, ou un cation organo-métallique, 1 ≤ m ≤ 3 ;
- Q représente N, CR⁵, CCN ou CSO₂R⁵ ;
- Z¹ **et** Z² désignés ci-après par Zⁱ, représentent indépendamment les uns des autres =O, =NC≡N, =C(C≡N)₂. =NS(=Z)₂R⁶ ou =C[S(=Z)₂R⁶]₂;
- R¹, R², et R⁶, désignés ci-après par Rⁱ, représentent indépendamment les uns des autres Y, YO-, YS-, Y₂N- ou F ;
- Y représente un radical organique monovalent, ayant de 1 à 16 atomes de carbone, choisi parmi les radicaux alkyles, alkényles, oxa-alkyles, oxa-alkényles, aza-alkyles, aza-alkényles, aryles ou alkylaryles, perfluoroalkyles, ou parmi les radicaux obtenus à partir des radicaux précités par substitution, dans les chaînes et / ou la partie aromatique, par des hétéroatomes tels que les halogènes, l'oxygène, l'azote, le soufre, le phosphore, étant entendu que **si** le soufre ou le phosphore sont présents, ils peuvent éventuellement être liés à des atomes d'oxygène ou d'azote substitués, ou bien Y est une unité de répétition d'une trame polymérique ;
- dans les formules (VIIb), (VIIc) (VIIe), (VIIf) et (VIIg), R_{f} représente un radical perfluoroalkyle ;
- dans les formules (VIIb) et (VIIe), CₙH₂ₙ₊₁ réprésente un radical alkyle ;
- dans les formules (VIIc) et (VIIf), Y', Y" et Y" ont la même définition que Y ;
- dans la formule (VIIa), R'⁶ est choisi dans le même groupe que R⁶ .
- dans la formule (VIId), R⁶ est différent de R¹.

2. Utilisation d'un composé ionique en tant que catalyseur selon la revendication 1, **caractérisée en ce que** le cation M^{m+} est un cation métallique choisi parmi K⁺, Li⁺, Mg⁺⁺, Ca⁺⁺ ou Ba⁺⁺, Cu⁺⁺, Zn⁺⁺, Fe⁺⁺ ou Re⁺⁺⁺.

3. Utilisation d'un composé ionique en tant que catalyseur selon la revendication 1, **caractérisée en ce que** le cation M^{m+} est un cation onium choisi dans le groupe constitué par les ions ammonium, les ions guanidinium, les ions amidinium, les ions pyridinium, les ions imidazolium, les ions imidazolinium, les ions triazolium, les ions sulfonium, les ions iodonium, les ions phosphonium, le nitrosyle et NO₂⁺.

4. Utilisation d'un composé ionique en tant que catalyseur selon la revendication 1, **caractérisée en ce que** le cation M^{m+} est un cation organo-métallique choisi parmi les ions métallocénium, les cations métalliques coordonnés par des atomes tels que O, S, Se N, P ou As et portés par des molécules organiques, les groupes trialkyl-silyle, tétraalkyl-germanyle et dialkyl-stannyle dans lesquels les radicaux alkyles ont de 1 à 10 atomes de carbone.

5. Utilisation d'un composé ionique en tant que catalyseur selon la revendication 1, **caractérisée en ce que** les radicaux R¹ et R² sont choisis indépendamment l'un de l'autre parmi les radicaux perfluoroalkyle, les radicaux alkyle, les radicaux alkényle, les radicaux dialkylamino et les radicaux styrényle.

6. Utilisation d'un composé ionique en tant que catalyseur selon la revendication **5**, **caractérisée en ce que** les radicaux perfluoroalkyle ont de 1 à 6 atomes de carbone, les radicaux alkyle ont de 1 à 8 atome de carbone, les radicaux alkényle ont de 2 à 18 atomes de carbone, les radicaux alkyle des groupements dialkylamino ont de 1 à 18 atomes de carbone.

7. Utilisation d'un composé ionique en tant que catalyseur selon la revendication **1**, **caractérisée en ce que** R¹ et R⁶ représentent indépendamment l'un de l'autre un radical choisi parmi F, CF₃, C₂F₅, C₄F₉, C₆F₁₃ et C₈F₁₇, et R² et R⁵ représentent indépendamment l'un de l'autre un alkyle, un aryle, un alkylaryle ou un dialkylamino ayant de 1 à 20 atomes de carbone.

8. Utilisation d'un composé ionique en tant que catalyseur selon l'une des revendications 1 à 7, pour une réaction de polymérisation ou de réticulation de monomères ou de prépolymères capables de réagir par voie cationique, ledit composé ionique agissant comme photoinitiateur source d'acide catalysant la réaction.

9. Utilisation d'un composé ionique en tant que catalyseur selon **la revendication 1**, pour la préparation de composés organiques optiquement actifs ou pour les réactions de polymérisation énantiosélectives, **ledit composé répondant à la formule (VIId).**

## Claims

1. Use of an ionic compound as the catalyst for a polymerization reaction, a condensation reaction, an addition or elimination reaction, an oxidation or reduction reaction, a solvolysis, a Friedel-Crafts reaction or a Diels-Alder reaction, **characterized in that** said ionic compound has one of the formulae 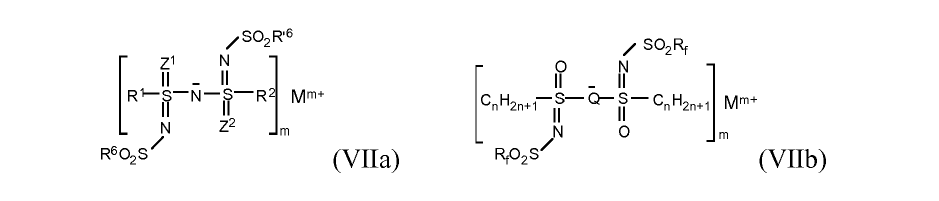 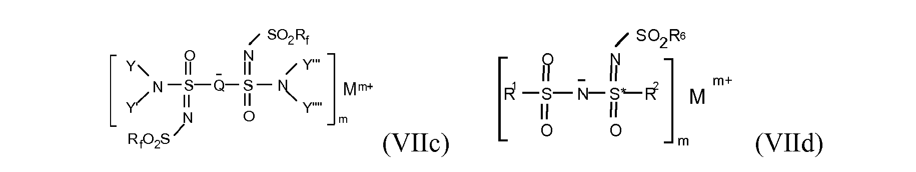 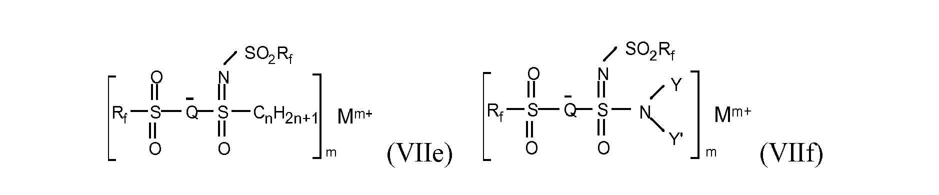 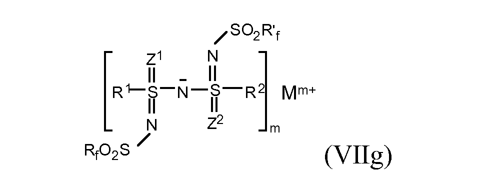 in which :
- M^{m+} is a proton or a metal cation having the valency m, chosen from ions of alkali metals, of alkaline-earth metals, of transition metals or of rare-earth metals, or an organic onium cation or an organometallic cation, 1 ≤ m ≤ 3;
- Q represents N, CR⁵, CCN or CSO₂R⁵;
- Z¹ and Z², denoted below by Zⁱ, represent, independently of each other, O, NC≡N, C(C≡N)₂, NS(=Z)₂R⁶ or C[S(=Z)₂R⁶]² ,;
- R¹, R², and R⁶, denoted below by R¹, represent, independently of each other, Y, YO-, YS-, Y₂N- or F;
- Y represents a monovalent organic radical chosen from alkyl, alkenyl, oxaalkyl, oxaalkenyl, azaalkyl, azaalkenyl, aryl, alkylaryl or perfluoroalkyl radicals, or from the radicals obtained from the abovementioned radicals by substitution, in the chains and/or the aromatic part, with hetero atoms chosen from halogens, oxygen, nitrogen, sulfur or phosphorus; with the proviso that if said heteroatom is sulfur or phosphorus, said sulfur or phosphorus atom can optionally be in the form of a -SO- group, a -SO₂ group or a >PO- group, or alternatively Y is a repeating unit of a polymeric backbone,
- in formulae (VIIb), (VIIc) (VIIe), (VIIf) and (VIIg), R_{f} represents a perfluoroalkyl radical;
- in formulae (VIIb) and (VIIe), CₙH₂ₙ₊₁ represents an alkyl radical;
- in formulae (VIIc) and (VIIf), Y', Y" and Y"' have the same meaning as Y ;
- in formulae (VIIa), R'⁶ is selected from the same group as R⁶,
- in formulae (VIId), R⁶ is different from R¹.

2. Use of an ionic compound as the catalyst according to Claim 1, **characterized in that** the cation M^{m+} is a metal cation chosen from K⁺, Li⁺, Mg⁺⁺, Ca⁺⁺ or Ba⁺⁺, Cu⁺⁺, Zn⁺⁺, Fe⁺⁺ or Re⁺⁺⁺.

3. Use of an ionic compound as the catalyst according to Claim 1, **characterized in that** the cation M^{m+} is an onium cation chosen from the group consisting of ammonium ions, guanidinium ions, amidinium ions, pyridinium ions, imidazolium ions, imidazolinium ions, triazolium ions, sulfonium ions, iodonium ions, phosphonium ions, nitrosyl and NO₂⁺.

4. Use of an ionic compound as the catalyst according to Claim 1, **characterized in that** the cation M^{m+} is an organometallic cation chosen from metallocenium ions, metal cations coordinated by O, S, Se, N, P or As atoms and borne by organic molecules, trialkylsilyl, tetraalkylgermanyl or dialkylstannyl groups in which the alkyl radicals contain from 1 to 10 carbon atoms.

5. Use of an ionic compound as the catalyst according to Claim 1, **characterized in that** the radicals R¹ and R² are chosen, independently of each other, from perfluoroalkyl radicals, alkyl radicals, alkenyl radicals, dialkylamino groups, and styrenyl radicals.

6. Use of an ionic compound as the catalyst according to Claim 5, **characterized in that** the perfluoroalkyl radicals contain from 1 to 6 carbon atoms, the alkyl radicals contain from 1 to 8 carbon atoms, the alkenyl radicals contain from 2 to 18 carbon atoms, the alkyl radicals in the dialkylamino groups contain from 1 to 18 carbon atoms.

7. Use of an ionic compound as the catalyst according to Claim 1, wherein R¹ and R⁶ represent, independently of each other, a radical chosen from F, CF₃, C₂F₅, C₄F₉, C₆F₁₃ or C₈F₁₇, and R² and R⁵ represent, independently of each other, an alkyl, an aryl, an arylalkyl or a dialkylamino containing from 1 to 20 carbon atoms.

8. Use of an ionic compound as the catalyst according to any of Claims 1 to 7, in a reaction of polymerizing or crosslinking monomers or prepolymers capable of reacting cationically, said compound acting as a photoinitiating source of acid for catalyzing the reaction.

9. Use of an ionic compound as the catalyst according to Claim 1, for preparing an optically active organic compound, or for enantioselective polymerisation reactions, said compound having formula (VIId).

## Patentansprüche

1. Verwendung einer ionischen Verbindung als Katalysator für eine Polymerisationsreaktion, eine Kondensationsreaktion, eine Additions- oder Eliminationsreaktion, eine Oxidations- oder Reduktionsreaktion, eine Solvolyse, eine Friedel-Crafts-Reaktion oder eine Diels-Alder-Reaktion, **dadurch gekennzeichnet, dass** die ionische Verbindung eine der folgenden Formeln hat: 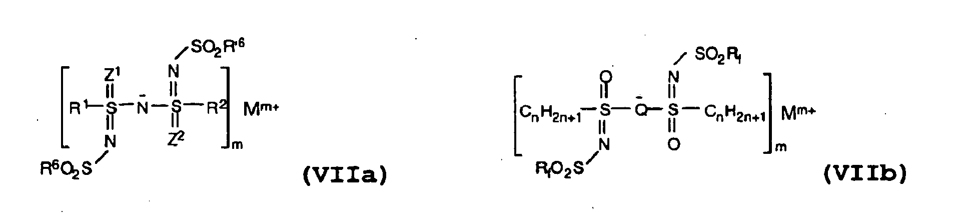 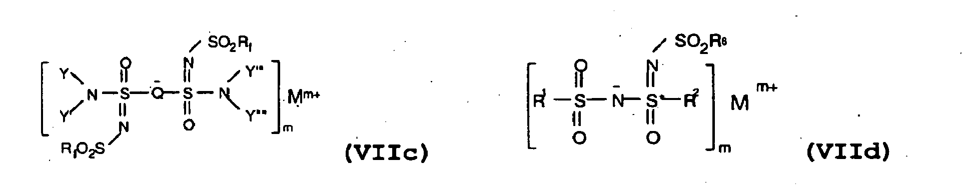 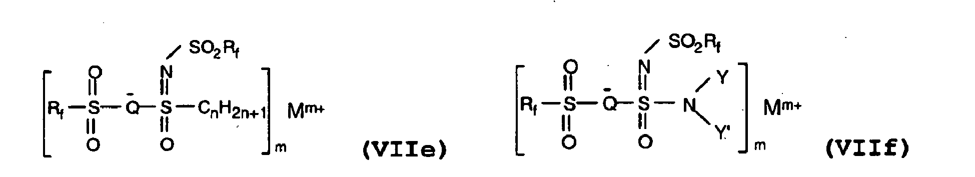 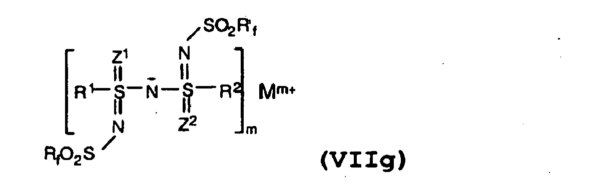 worin:
- M^{m+} ein Proton oder ein Metallkation mit der Wertigkeit m, ausgewählt aus Alkalimetall-, Erdalkalimetall-, Übergangsmetall- und Seltenerdelementionen, oder ein organisches Oniumkation oder ein organometallisches Kation ist, wobei gilt: 1 ≤ m ≤ 3;
- Q für N, CR⁵, CCN oder CSO₂R⁵ steht;
- Z¹ und Z², die nachstehend als Zⁱ bezeichnet werden, unabhängig voneinander für =O, =NC≡N, =C(C≡N)₂, =NS(=Z)₂R⁶ oder =C[S(=Z)₂R⁶]₂ stehen;
- R¹, R² und R⁶, die nachstehend als Rⁱ bezeichnet werden, jeweils unabhängig voneinander für Y, YO-, YS-, Y₂N- oder F stehen;
- Y für einen einwertigen organischen Rest mit 1 bis 16 Kohlenstoffatomen steht, der aus Alkyl-, Alkenyl-, Oxaalkyl-, Oxaalkenyl-, Azaalkyl-, Azaalkenyl-, Aryl- oder Alkylaryl- und Perfluoralkylresten oder aus Resten ausgewählt ist, die durch Substitution der oben genannten Reste in den Ketten und/oder dem aromatischen Abschnitt durch Heteroatome, wie z.B. Halogene, Sauerstoff, Stickstoff, Schwefel, Phosphor, erhalten werden, wobei es sich versteht, dass, wenn Schwefel oder Phosphor vorhanden sind, diese gegebenenfalls an substituierte Sauerstoff- oder Stickstoffatome gebunden sein können, oder aber Y eine Grundeinheit eines Polymergerüsts ist;
- R_{f} in den Formeln (VIIb), (VIIc), (VIIe), (VIIf) und (VIIg) für einen Perfluoralkylrest steht;
- CₙH₂ₙ₊₁ in den Formeln (VIIb) und (VIIe) für einen Alkylrest steht;
- Y', Y" und Y"' in den Formeln (VIIc) und (VIIf) wie Y definiert sind;
- R'⁶ in der Formel (VIIa) aus derselben Gruppe wie R⁶ ausgewählt ist;
- sich R⁶ in der Formel (VIId) von R¹ unterscheidet.

2. Verwendung einer ionischen Verbindung als Katalysator nach Anspruch 1, **dadurch gekennzeichnet, dass** das Kation M^{m+} ein Metallkation ist, das aus K⁺, Li⁺, Mg⁺⁺, Ca⁺⁺ oder Ba⁺⁺, Cu⁺⁺, Zn⁺⁺, Fe⁺⁺ oder Re⁺⁺⁺ ausgewählt ist.

3. Verwendung einer ionischen Verbindung als Katalysator nach Anspruch 1, **dadurch gekennzeichnet, dass** das Kation M^{m+} ein Oniumkation ist, das aus der aus Ammoniumionen, Guanidinionen, Amidinionen, Pyridinionen, Imidazolionen, Imidazolinionen, Triazolionen, Sulfonionen, Iodoniumionen, Phosphoniumionen, Nitrosyl und NO₂⁺ bestehenden Gruppe ausgewählt ist.

4. Verwendung einer ionischen Verbindung als Katalysator nach Anspruch 1, **dadurch gekennzeichnet, dass** das Kation M^{m+} ein organometallisches Kation ist, das aus Metallocenionen, mit Atomen wie O, S, Se, N, P oder As koordinierten und von organischen Molekülen getragenen Metallkationen, Trialkylsilyl-, Tetraalkylgermanyl- und Dialkylstannylgruppen, worin die Alkylreste 1 bis 10 Kohlenstoffatome aufweisen, ausgewählt ist.

5. Verwendung einer ionischen Verbindung als Katalysator nach Anspruch 1, **dadurch gekennzeichnet, dass** die Reste R¹ und R² unabhängig voneinander aus Perfluoralkylresten, Alkylresten, Alkenylresten, Dialkylaminoresten und Styrenylresten ausgewählt sind.

6. Verwendung einer ionischen Verbindung als Katalysator nach Anspruch 5, **dadurch gekennzeichnet, dass** die Perfluoralkylreste 1 bis 6 Kohlenstoffatome aufweisen, die Alkylreste 1 bis 8 Kohlenstoffatome aufweisen, die Alkenylreste 2 bis 18 Kohlenstoffatome aufweisen und die Alkylreste der Dialkylaminogruppen 1 bis 18 Kohlenstoffatome aufweisen.

7. Verwendung einer ionischen Verbindung als Katalysator nach Anspruch 1, **dadurch gekennzeichnet, dass** R¹ und R⁶ unabhängig voneinander für einen aus F, CF₃, C₂F₅, C₄F₉, C₆F₁₃ und C₈F₁₇ ausgewählten Rest stehen und R² und R⁵ unabhängig voneinander für ein Alkyl, ein Aryl, ein Alkylaryl oder ein Dialkylamino mit 1 bis 20 Kohlenstoffatomen stehen.

8. Verwendung einer ionischen Verbindung als Katalysator nach einem der Ansprüche 1 bis 7 für eine Polymerisationsreaktion oder eine Vernetzungsreaktion von Monomeren oder Präpolymeren, die auf kationischem Weg umgesetzt werden können, wobei die ionische Verbindung als photoinitiierende Säurequelle dient, die die Reaktion katalysiert.

9. Verwendung einer ionischen Verbindung als Katalysator nach Anspruch 1 zur Herstellung von optisch aktiven organischen Verbindungen oder für enantioselektive Polymerisationsreaktionen, wobei die Verbindung die Formel (VIId) hat.
